Europäisches Patentamt

European Patent Office

Office européen des brevets

(11) Veröffentlichungsnummer: **0 659 716 A1**

## (12) EUROPÄISCHE PATENTANMELDUNG

(21) Anmeldenummer: **94119206.4**

(22) Anmeldetag: **06.12.94**

(51) Int. Cl.6: **C07B 63/00**, C11D 1/00,
//C07C231/24,C07C303/44,
C07C213/10,C07D233/00

(30) Priorität: **24.12.93 DE 4344644**

(43) Veröffentlichungstag der Anmeldung:
**28.06.95 Patentblatt 95/26**

(84) Benannte Vertragsstaaten:
**DE ES FR GB**

(71) Anmelder: **Witco Surfactants GmbH**
**Industriegebiet West**
**D-36396 Steinau an der Strasse (DE)**

(72) Erfinder: **Hiller, Dr. Günter**
**Mistelweg 5**
**D-36391 Sinntal-Sannerz (DE)**
Erfinder: **Lossow, Michael**
**Am Schafhof 2**
**D-36396 Steinau an der Strasse (DE)**
Erfinder: **Schmidt, Dieter**
**An der Häsel 1**
**D-36396 Steinau an der Strasse (DE)**

(54) **Verfahren zur Herstellung hellfarbiger Tenside.**

(57) Die Erfindung betrifft ein Verfahren zur Herstellung hellfarbiger Tenside durch Bestrahlung von flüssigen, gegebenenfalls Lösungsmittel enthaltenden Tensiden mit UV-Strahlung.

Rank Xerox (UK) Business Services
(3.10/3.09/3.3.4)

EP 0 659 716 A1

Gegenstand der Erfindung ist ein verfahren zur Herstellung hellfarbiger Tenside durch Bestrahlung von flüssigen, gegebenenfalls Lösungsmittel enthaltenden Tensiden mit UV-Strahlung.

Auf dem Gebiet der Haar- und Körperreinigung, der Haar- und Körperpflege, der Textilreinigung werden je nach Anwendungsgebiet und Zweck anionische, kationische, nichtionische und amphotere Tenside allein oder in Mischung in den entsprechenden Rezepturen eingesetzt. Eine Definition und Klassifizierung dieser Tenside wird im Tensid-Taschenbuch, Carl Hanser Verlag, München, Wien, 1981, Seite 2 ff gegeben. Hier werden auch zu den für die jeweiligen Anwendungsgebiete gebräuchlichen Tensiden und deren Anforderungsprofile Aussagen gemacht.

Eines dieser Kriterien ist die Farbe. Helle, möglichst wasserklare Produkte sind danach ein primäres Spezifikationsmerkmal, um den Formulierern eine möglichst freie Hand bei der Endkonfektionierung zu erlauben.

Aufgrund der verwendeten Rohstoffe und Strukturen neigen tensidische Verbindungen während der Produktion und Lagerung zur Verfärbung.

Üblicherweise werden derartige tensidische Verbindungen daher nach Fertigstellung der Synthese durch Zusatz von anorganischen und organischen Hilfsstoffe nachgebleicht. Dies können z. B. sein $H_2O_2$, Sulfit, Natriumperborat, Natriumborhydrid, Hypochlorit, Percarbonate. Der Nachteil dieser Verfahren liegt betrieblicherseits in einem weiteren Behandlungsschritt. Der Nachteil aus Sicht des Anwenders liegt darin, daß durch diese Art der Nachbehandlung die Aktivkomponente zusätzlich mit Fremdstoffen befrachtet wird, die mitunter - so z. B. in kosmetischen Präparaten hoher Reinheit - unerwünscht sind. Bei Gehalten über 5 - 10 ppm besteht weiterhin immer die Gefahr der unkontrollierten Weiterreaktion mit Duftstoffen, Farbstoffen, Stabilisatoren, UV-Filtern und sonstigen Inhaltsstoffen.

Fein- und Buntwaschmittel, welche zum Waschen empfindlicher Textilien verwendet werden, sollten insbesondere dann frei von peroxidischen Bestandteilen sein, wenn sauerstoffempfindliche Färbungen vorliegen oder wenn die Gefahr von Farbverschiebungen vorliegt. Bei der chemischen Aufhellung ist ein Restgehalt an peroxidischen Bestandteilen aber unvermeidbar.

Um den nach dem Bleichvorgang vorhandenen Restgehalt an den jeweils verwendeten chemischen Hilfsstoffen weitgehend zu mindern, werden verschiedene Verfahren angewandt: Peroxide werden üblicherweise thermisch zerstört, was aufgrund der dabei auftretenden thermischen und oxidativen Belastung den vorher erzielten Bleicheffekt teilweise wieder aufheben kann; Borhydride können durch Säurebehandlung zerstört werden, was die unerwünschte Belastung der Tenside mit Salzen erhöht; die hydrierende Nachbehandlung in Gegenwart von Edelmetallkatalysatoren ist apparativ aufwendig und teuer.

Aufgabe der vorliegenden Erfindung war es daher, ein Verfahren zur Herstellung hellfarbiger Tenside zu finden, welches ohne Mitverwendung organischer und anorganischer Hilfsstoffe auskommt und allgemein anwendbar ist.

Diese Aufgabe wird dadurch gelöst, daß die flüssigen Tenside oder Tensidlösungen über einen ausreichend langen Zeitraum einer UV-Strahlung ausgesetzt werden.

Gegenstand der Erfindung ist daher ein Verfahren zur Herstellung hellfarbiger Tenside, welches dadurch gekennzeichnet ist, daß die Tenside oder Tensidlösungen über einen Zeitraum von 1 - 4 Stunden, vorzugsweise 1,5 - 2,5 Stunden, einer Strahlung im Bereich von ca. 200 - ca. 700 nm, vorzugsweise 250 - 650 nm, ausgesetzt werden.

Mit dem erfindungsgemäßen Verfahren lassen sich überraschenderweise Farbverbesserungen erzielen, die deutlich über denen liegen, welche mit den bekannten Verfahren unter Mitverwendung chemischer Hilfsstoffe liegen und welche in Einzelfällen von diesen Hilfsstoffen überhaupt nicht geleistet werden können.

Die erfindungsgemäß verwendeten Reaktionsbehälter sind handelsübliche Produkte, welche u. a. als UV-Tauchlampenreaktoren, ausgerüstet mit Quecksilberhochdruckbrennern, als UV-Strahlungsquellen von den Herstellerfirmen unter ihren jeweiligen Typenbezeichnungen angeboten werden.

Diese Quecksilberhochdruckbrenner emittieren Strahlung in einem Spektrum, welches vom kurzwelligen UV-Bereich von ca. 200 nm bis in den sichtbaren Bereich reicht.

Erfindungsgemäß bevorzugt werden UV-Strahlungsquellen, deren Spektrum im Bereich von ca. 200 bis ca. 700 nm, vorzugsweise im Bereich von 250 bis 650 nm, liegen.

Die Bestrahlungsdauer variiert Je nach Tensid zwischen 1 bis 3 Stunden, wobei die besten Ergebnisse bei ca. 2 Stunden erzielt werden. Diese Zeiten können sich bei Verwendung von UV-Strahlen anderer Bauart und deren spektraler Energieverteilung naturgemäß verschieben.

Die Tenside, welche mit dem erfindungsgemäßen Verfahren aufgehellt werden können, sind die auf den jeweiligen Anwendungsgebieten üblichen meist wässrigen Lösungen von anionischen, kationischen, nichtionischen, amphoteren und zwitterionischen Verbindungen wie beispielsweise Carboxylate, Succinate, Sarcoside, Amido-ethersulfate, Amidoglycinate, Aminoglycinate, Ethersulfate, Sulfosuccinate, primäre, sekundäre,

2

tertiäre und quarternäre Alkyl- und Arylammoniumsalze, Imidazoliniumsalze, Imidazolincarboxylate, Betaine, Aminocarbonsäuren, Alkyl- und Arylpolyglykolether, Alkyl- und Arylpolyglykolester, Glycoside. Für einen umfassenden Überblick über die erfindungsgemäß aufhellbaren Tenside wird auf die einschlägige Fachliteratur verwiesen wie Kirk-Othmer, Encyclopedia of Chemical Technology, 3rd Edition, 1983, Vol 22, 332-432; Ullmann's Encyclopedia of Industrial Chemistry, Vol A8, 1987, 315-448.

Diese Tenside können in reiner Form oder vorzugsweise als wässrige Lösungen eingesetzt werden. Hierbei ist der Feststoffgehalt der Lösungen unkritisch und kann innerhalb weiter Grenzen schwanken, soweit die Viskosität der reinen Tenside/Tensidlösungen die Rührfähigkeit und damit die intensive Durchmischung während des Bestrahlungsvorganges nicht beeinträchtigt. Aus wirtschaftlichen Gründen werden die durch die Herstellungsprozesse vorgegebenen Konzentrationen unverändert beibehalten oder möglichst hochkonzentrierte Lösungen eingesetzt.

Soweit die Produkte thermisch belastbar sind, kann zur Viskositätsminderung auch die Temperatur soweit erhöht werden, aber nur soweit, daß bei der anschließenden Bestrahlung keine unerwünschten Nebenreaktionen in störendem Maße auftreten.

Versuchsdurchführung

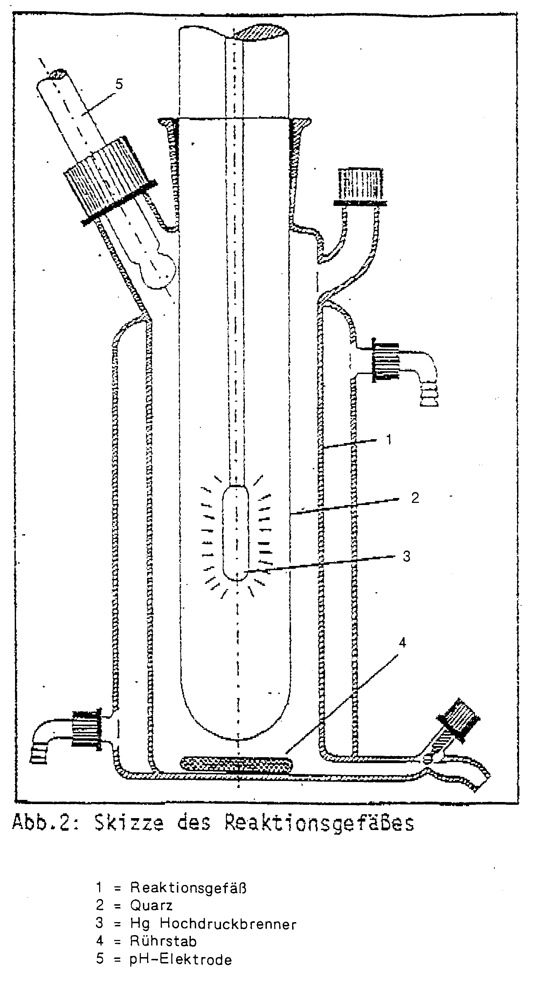

In einem UV-Tauchlampenreaktor aus Glas und einem Reaktorvolumen von ca. 450 ml (bei eingetauchter UV-Lampe), versehen mit einem Kühlmantel und ausgerüstet mit einem UV-Quecksilberdampf-Hochdruckbrenner (vergl. Abbildung 2) wurden jeweils 250 ml der wässrigen Lösung des Versuchsproduktes in seiner handelsüblichen Form eingefüllt und bei 25°C (AM 2 C NM und WE 18 50 °C) unter intensivem Rühren 2 Stunden bestrahlt. Danach wurde das Produkt abgelassen und die Farbzahl nach der angegebenen Methode bestimmt.

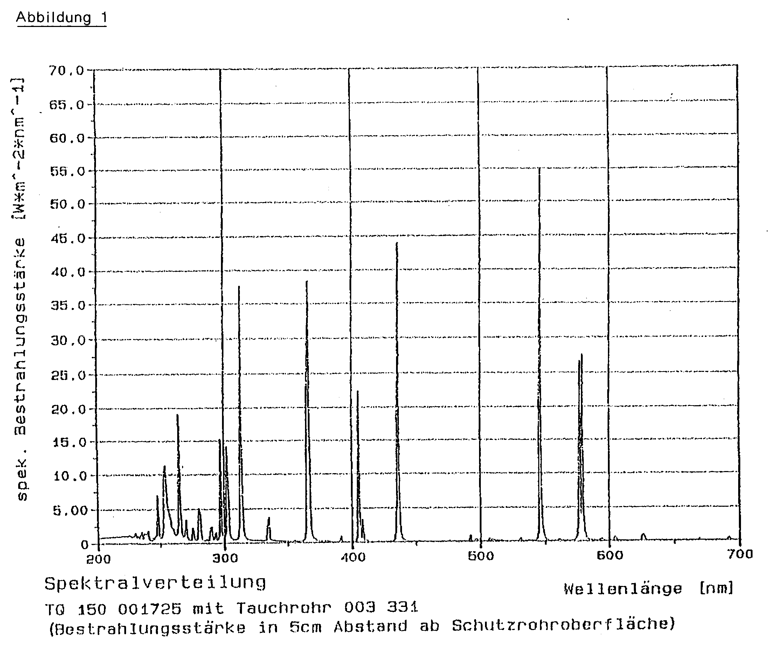

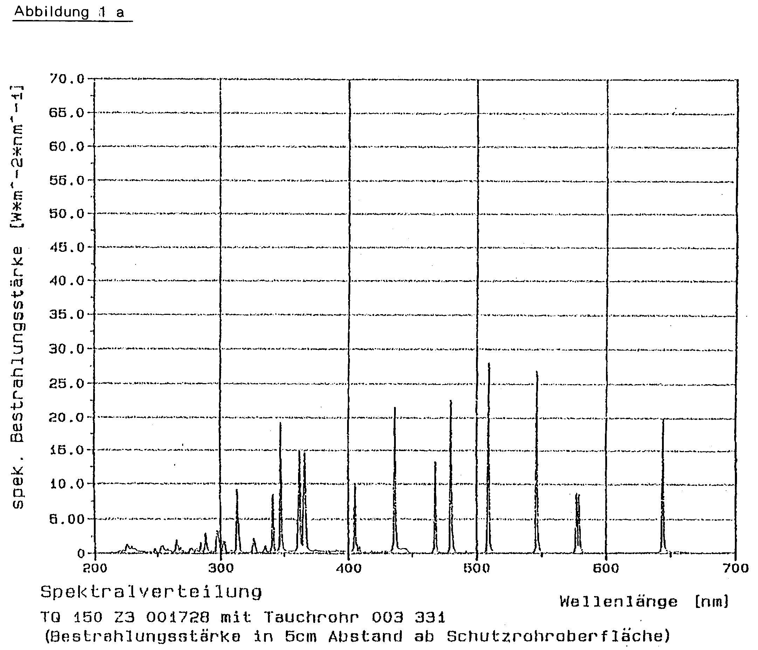

Die Beispiele der Tabelle 1 wurden mit einem UV-Quecksilberdampf-Hochdruckbrennerdes Typs TQ 150 , die der Tabelle 2 des Typs TQ 150 Z3 (Fa. Heraeus Instruments GmbH) durchgeführt. Die Spektralverteilung dieser Strahler ist in den Abbildungen 1 und 1 a gegeben.

In der Tabelle bedeuten:

FL = Farbe Lovibond:

Prinzip:

Die Farbe der Probe wird durch Messung der optischen Dichte an zwei Punkten des Spektrums vermessen.

Geräte:

- Automatisches Lovibond Tintometer LICO 200
- Küvetten 5 1/4 Zoll

Durchführung:

Die gefüllte Küvette wird in das Gerät gesetzt, Messung und Eichung erfolgt nach Bedienungsanleitung des Geräts. Die Farbe kann direkt im Display abgelesen werden.

Hersteller:

Dr. Lange GmbH, Berlin.
Literatur:    DGF*:C-IV 4b
                   * Deutsche Gesellschaft für Fettwissenschaften

FH = Farbe Hazen

Prinzip:

Die Farbe der Probe wird mit Standardfarbscheiben visuell verglichen.

Geräte:

- BOH Lovibond Nessleriser MK 3
- 50ml Nessler-Zylinder
- Farbscheiben Hazen 28-411-4

Durchführung:

Die Probe wird in den Nessler-Zylinder gesetzt. Die Farbscheiben werden so lange gedreht, bis Farbgleichheit mit der Probe erreicht ist. Das Ergebnis kann direkt in Hazen-Einheit abgelesen werden.

Literatur: DIN 53409.

FG = Farbe Gardner

Prinzip:

Die Farbe der Probe wird mit Standardfarbscheiben visuell gemessen.

Geräte:

- Reagenzgläser 12 x 100 mm
- Lovibond 3-Feld-Komperator mit Standard-Weißlichtquelle
- Farbscheiben Gardner 1963

Durchführung:

Die Probe wird in ein Reagenzglas gegeben und in das Gerät gesetzt. Die beiden Farbstandardscheiben werden so lange gedreht, bis die Farbe der Probe zwischen den Vergleichsmusters liegt oder mit diesen gleich ist. Das Ergebnis kann direkt in Gardner-Einheiten abgelesen werden.

| Produkt | Bezeichnung | Feststoffgehalt Gew.-% |
|---|---|---|
| REWOTERIC® AM B 13 | Cocoamidopropyl Betain | 35 |
| REWOTERIC® AM B 45 | Cocoamidopropyl Betain | 45 |
| REWOTERIC® AM 2 CNM | Di-Natrium-Cocoamphodiacetat | 51 |
| REWOTERIC® AM KSF 40 | Natrium-Cocoamphopropionate | 41 |
| REWOPOL® V 3270 | Mischung aus AMB 13 und Natriumlaurylethersulfat | 28 |
| REWOPOL® SB FA 30 | Disodium Laureth Sulfosuccinate | 41 |
| REWOQUAT® WE 18 | Di(talgcarboxyethyl)-hydroxyethyl-methyl-ammonium-sulfat | 91 |
| REWOQUAT® W 3690 | Dioleylimidazolinmethosulfat | 75 |
| REWOTERIC®, REWOPOL®, REWOQUAT® = Warenzeichen der REWO Chemische Werke GmbH | | |

## Tabelle 1

| Produkt | Meß-zelle Zoll | Ausgangsfarbe | | | Farbe Endprodukt | |
|---|---|---|---|---|---|---|
| | | Methode | Anteil | | Anteil | |
| | | | Gelb | Rot | Gelb | Rot |
| REWOTERIC® AM B 13 | 5 1/4 | FL | 5,3 | 0,7 | 1,5 | 0,2 |
| REWOTERIC® AM B 45 | | FH | 110 | | 39 | |
| REWOTERIC® AM 2 C NM | 1 | FL | 2,2  2,7 | 0,5 | 0,8  0,8 | 0,2 |
| REWOTERIC® AM KSF 40 | | FG | 5,1 | | 4,6 | |
| REWOPOL® V 3270 | 1 | FH FL | 65  0,32 | 0,1 | 32  0,1 | 0,0 |
| REWOPOL® SB FA 30 | 5 1/4 | FH FL | 23  0,6 | 0,1 | 21  0,6 | 0,0 |
| REWOQUAT® WE 18 | 5 1/4 | FG FL | 1,3  8,9 | 2,7 | 0,6  5,3 | 1,4 |
| REWOQUAT® W 3690 | 1 | FL | 9,4 | 1,4 | 8,2 | 1,4 |

Tabelle 2

| Produkt | Meß-zelle Zoll | Ausgangsfarbe | | | Farbe Endprodukt | |
|---|---|---|---|---|---|---|
| | | Methode | Anteil Gelb | Rot | Anteil Gelb | Rot |
| REWOTERIC® AM B 13 | 5 1/4 | FL | 4,6 | 0,7 | 0,8 | 0,6 |
| REWOTERIC® AM B 45 | | FH | 88 | | 31 | |
| REWOTERIC® AM 2 C NM | 1 | FG FL | 2,9 2,0 | 0,5 | 1,3 1,1 | 0,2 |
| REWOPOL® V 3270 | 1 | FH FL | 64 0,2 | 0,6 | 59 0,3 | 0,1 |
| REWOQUAT® WE 18 | 5 1/4 | FG FL | 1,4 10 | 2,9 | 0,7 6,1 | 1,3 |
| REWOQUAT® W 3690 | 1 | FL | 18 | 2,9 | 16 | 2,6 |

**Patentansprüche**

1. Verfahren zur Herstellung hellfarbiger Tenside, dadurch gekennzeichnet, daß die Tenside oder Tensidlösungen über einen Zeitraum von 1 - 4 Stunden einer Strahlung im Bereich von ca. 200 - ca. 700 nm ausgesetzt werden.

Abbildung 1

Spektralverteilung

TQ 150 001725 mit Tauchrohr 003 331
(Bestrahlungsstärke in 5cm Abstand ab Schutzrohroberfläche)

Wellenlänge [nm]

spek. Bestrahlungsstärke [W*m^-2*nm^-1]

Abbildung 1 a

Spektralverteilung

Wellenlänge [nm]

TQ 150 Z3 001728 mit Tauchrohr 003 331
(Bestrahlungsstärke in 5cm Abstand ab Schutzrohroberfläche)

EP 0 659 716 A1

Abb.2: Skizze des Reaktionsgefäßes

1 = Reaktionsgefäß
2 = Quarz
3 = Hg Hochdruckbrenner
4 = Rührstab
5 = pH-Elektrode

**Europäisches Patentamt**

**EUROPÄISCHER RECHERCHENBERICHT**

Nummer der Anmeldung

EP 94 11 9206

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE-B-12 80 848 (FARBWERKE HOECHST) <br> * das ganze Dokument * <br> --- | 1 | C07B63/00 <br> C11D1/00 <br> //C07C231/24, <br> C07C303/44, <br> C07C213/10, <br> C07D233/00 |
| X | DE-A-20 00 029 (PROCTER & GAMBLE) <br> * Ansprüche; Beispiel 2 * <br> --- | 1 | |
| X | DE-A-19 55 168 (PROCTER & GAMBLE) <br> * Ansprüche; Beispiele * <br> --- | 1 | |
| X | DE-A-24 26 187 (BASF) <br> * Seite 6 - Seite 7; Anspruch * <br> ----- | 1 | |

**RECHERCHIERTE SACHGEBIETE (Int.Cl.6)**

C07B
C07C

Der vorliegende Recherchenbericht wurde für alle Patentansprüche erstellt

| Recherchenort | Abschlußdatum der Recherche | Prüfer |
|---|---|---|
| DEN HAAG | 23. März 1995 | Wright, M |